# NEW EUROPEAN PATENT SPECIFICATION

(11) **EP 2 094 217 B2**
(45) Date of publication and mention of the opposition decision: **10.10.2018**
(45) Mention of the grant of the patent: 30.03.2016
(21) Application number: 07866620.3
(22) Date of filing: 23.11.2007
(51) Int. Cl.: A61F 13/20, A61F 13/42

(54) **CATAMENIAL AND SANITARY TAMPONS**
TAMPON
TAMPONS CATAMÉNIAUX ET SANITAIRES

(30) Priority: 24.11.2006 US 860798 P; 12.01.2007 US 880023 P; 01.02.2007 US 898710 P; 30.03.2007 US 920776 P; 02.04.2007 US 907411 P; 29.06.2007 US 929490 P
(43) Date of publication of application: 02.09.2009
(60) Divisional application: 11183229.1
(73) Proprietor: Essity Hygiene and Health Aktiebolag, 405 03 Göteborg (SE)
(72) Inventor: Chaffringeon, Bernard, Dubai UAE (AE)
(74) Representative: Zacco Sweden AB
(86) International application number: PCT/IB2007/004343
(87) International publication number: WO 2008/062322

(56) References cited:
- EP-A- 0 988 847
- EP-A- 1 064 901
- WO-A-96/00552
- WO-A-03/002049
- WO-A-2004/021943
- WO-A-2006/005069
- FR-A- 2 803 208
- US-A- 4 335 721
- US-A- 4 787 895

## Description

This disclosure is generally related to the field of sanitary tampons and to devices permitting absorption of the body's natural waste fluids.

In particular the present invention relates to a sanitary tampon with the features of the introductory part of claim 1, to a sanitary tampon with the features according to the introductory part of claims 10, 12 or 20, as well as to a method for manufacturing a sanitary tampon according to claim 28.

Many sanitary articles exist, especially for absorbing menstrual fluids, in particular catamenial and/or sanitary tampons made of absorbent material in the form of sheets and/or bands that are folded and/or rolled up, using techniques well known to persons skilled in the art, in order to form tampons that are inserted, with or without applicators, into the vaginas of the users.

However, although there are many models made of various materials and with absorption capacities that vary according to the particular requirements and losses, problems associated with leaking and/or soiling still exist that are due either to absorption capacities that are too low or to problems of volume, or even poor adherence to the vaginal wall, or a drainage phenomenon due in particular to the withdrawal devices, namely the threads and/or strings designed to remove the used tampon.

A problem also exists at the moment of withdrawal when the withdrawal devices, namely the threads and/or strings designed to remove the used tampon, and the vaginal wall can exert a pressure on the tampons and cause drying, which leads to a flow of fluid that soils the user's hands. Moreover, given that the lower part of the tampon, also called the proximal part, is greatly enlarged, this part has a tendency to cause discomfort or injuries by rubbing on the vaginal wall, especially in the area of muscular narrowing of the vaginal wall.

Many devices have already been proposed. Patent application WO 2006/094753 A1 discloses a tampon with an impermeable zone formed by a plastic film and zones of variable absorption in order to avoid drying of the vaginal walls. US 3,693,622 A discloses tampons and other sanitary articles which, at their ends, comprise zones impregnated with fluid-repelling compositions.

The devices described in these documents nevertheless have numerous disadvantages, such as the possibility of the fluid-repelling compositions migrating into the whole of the tampon and/or the sanitary article after production, the possibility of the fluid-repelling compositions being transferred to the user's finger at the moment of insertion of the tampon, and especially the impossibility of limiting the drying effect during withdrawal. Moreover, the disclosed devices do not solve the problem of the shaping and adaptation of a tampon such that it better matches the shape of the vaginal walls.

US 7,060,057 A discloses tampons comprising nonabsorbent zones arranged on the outer surface of the tampon that permit increased comfort at the interface between the tampon and the vaginal walls.

US 7,097,638 A discloses tampons comprising zones that form reinforcements on the outer walls of the tampon in order to improve comfort while at the same time maintaining the absorption capacities of the tampons.

US 3,965,905 A discloses tampons formed of a plurality of absorbent product parts connected by a withdrawal cord.

These different devices, however, do not significantly improve absorption while at the same time improving comfort and retaining other important properties. The tampon described in US 3,965,905 A, for example, does not provide a tampon that is sufficiently cohesive to permit its insertion without an applicator.

US 2003/0097108 A describes a tampon comprising an absorbent compressed body whose outer face is largely covered by an absorbent shroud that forms a skirt beyond the proximal end of the body of the tampon.

US 2005/0096620 A describes a tampon obtained by rolling up a layer of absorbent material, the layer having an edge intended to constitute the proximal end after rolling, and being covered by an absorbent shroud before rolling.

US 4,211,225 A describes a tampon permitting improved comfort during its withdrawal. For this purpose, a first shroud and then a second shroud are attached to the body of the tampon and are fixed in the area of the distal end of the tampon. The first shroud is designed such that the frictional forces generated during withdrawal between the body of the tampon and the first shroud are less than the frictional forces between the second shroud and the surrounding tissues.

The sanitary tampon that forms the starting point of the invention (FR 2 803 208 A; US 2002/0173760 A1) comprises a body of absorbent material and a withdrawal thread fixed to the absorbent material of this body. The body of the tampon, as usual for every tampon, has a distal end, the front end when inserting the tampon into the vagina of a user, and an opposite proximal end, the rear end on application, where the withdrawal thread extends from the proximal end of the body so that the user may grab the withdrawal thread if necessary to pull and remove the tampon. The sanitary tampon of this prior art has its distal end covered with a solid covering of a hydrophobic and antiseptic substance that has a melting point slightly greater than 37 degrees centigrade

While the prior art sanitary tampon described above is successful in avoiding the risks of infection of the vagina by use of the hydrophobic substance that is also an antiseptic, it doesn't change the characteristics of the sanitary tampon as far as the absorption of absorbable fluids is concerned.

So the object of the present invention is to improve on a sanitary tampon as far as the characteristics when the body contacts an absorbable fluid is concerted.

The above mentioned object is met with a sanitary tampon of a first variant by means of the features of claim 1. Preferred modifications and improvements are the subject matter of dependent claims 2 to 9.

In a second variant of the sanitary tampon according to the invention the above mentioned object is met with a sanitary tampon with the features of claims 10, 12 or 20. Here preferred modifications and embodiments are the subject matter of dependent claims 11 and 13 to 19, and 21-25.

In a particular preferred version the tampon combines both variants of the invention according to claim 26.

The invention also relates to a method for manufacturing a sanitary tampon as described in claim 28. This method is improved by further embodiments and modifications according to the dependent claims 29 to 23.

In the first variant the sanitary tampon according to the invention comprises means for limiting the expansion and/or the absorption of at least one zone of the body of the tampon when the tampon comes into contact with a fluid that is to be absorbed.

The sanitary tampon according to the invention is configured so that the part in which the expansion and/or the absorption is limited is situated at or near to the proximal end of the body.

Thus, the control of the expansion and/or of the absorption of the body of the tampon in this zone permits formation, at this location, of a fluidic barrier that is more or less impervious with respect to the absorbed fluid. In some embodiments, the body of the tampon may comprise a plurality of zones formed by fluidic barriers.

The expansion and/or absorption are limited by a solid hydrophobic substance whose melting point is greater than 37 degrees centigrade, which partially or totally impregnates the absorbent material from which the tampon is made.

The disclosure is also directed to tampons having a withdrawal thread that is also at least partially impregnated with a solid hydrophobic substance whose melting point is greater than 37 degrees centigrade

By using a solid hydrophobic substance whose melting point is greater than 37 degrees centigrade, the zones in which the expansion and/or the absorption are limited are permanent, that is to say, the zones are nondeformable or are only minimally deformable, even when the tampon is saturated.

In the first variant, the hydrophobic substance is stearin.

In embodiments, the part in which the expansion and/or the absorption are limited is situated at a distance of more than 0.5 mm from the proximal end of the tampon.

In embodiments, the zone in which the expansion and/or the absorption is limited forms a spiral inside and outside the tampon.

In embodiments, the tampon additianally comprises another zone in which the expansion and/or the absorption are limited, situated halfway between the two ends of the tampon.

In embodiments, the tampon additionally comprises another part in which the expansion and/or the absorption are limited, forming a spiral outside the tampon. In such embodiments, and in contrast to the tampon described and shown in EP 1 383 453 A, the spiral shape obtained may be nondeformable or only minimally deformable, even after saturation of the tampon with a hydrophilic substance, and this spiral shape makes it possible to channel the flow and thus avoid flow of fluid along the vaginal walls, and also to increase the time of contact between the absorbent material and the flow, thus permitting improved absorption and a greater capacity of the tampon.

The tampons may be impregnated with the solid hydrophobic substance whose melting point is greater than 37 degrees centigrade in the melted state and before and/or after formation of the tampon. When the substance is applied before formation of the tampon, it is applied to the band before folding and/or compression and/or rolling of the band.

A zone formed by a line measuring several tenths of a millimeter to several millimeters is applied continuously or discontinuously to the front face and/or rear face of the band, optionally in several zones, and optionally nonsymmetrically. The zones of impregnation may be arranged on what will become the lower part, middle part, and/or the upper part of the tampon in order to create one or more reservoirs with these successive barriers.

The zones may be arranged in such a way that, after folding and/or compression of the band, a spiral shape is obtained that is nondeformable or only minimally deformable, even after saturation of the tampon with a hydrophilic substance. This spiral shape makes it possible to channel the flow, and thus avoid flow of fluid along the vaginal walls, and also to increase the time of contact between the absorbent material and the flow, thus improving absorption and capacity of the tampon.

In embodiments, the tampon comprises an impregnation zone in the form of an up-turned funnel which, by diverting the flow from the center of the tampon, makes it possible to avoid accumulation of fluid at the site of fixation of a withdrawal thread and to avoid leaks due to the presence of this withdrawal thread.

According to the secont variant of the sanitary tampon the zone in which expansion and/or absorption is limited is defined by at least one restriction member encircling a portion of the body of the tampon. The restriction member is configured to permanently restrict a diameter of the body of the tampon at said zone from expanding upon absorption of a fluid by the tampon. This zone is maintained in its initial shape when the other parts of the body of the tampon inflate under the effect of absorption of a fiuid.

The restriction member of the sanitary tampon according to the invention comprises at least one ring, washer, collar, or foldable restrictor band.

In embodiments, the restriction member comprises, on its outer face, at least one smooth zone that makes it possible to limit the friction during removal of the tampon, so as to avoid any injury.

Advantageously, the ring is formed with the protector of the tampon.

In a second version of the second variant, each restrictor band comprises at least one orifice near each of its two ends, the orifice having a diameter that is substantially equal to that of the compressed tampon. Where appropriate, the restrictor band may comprise at least a third orifice in the central part for passage of the withdrawal thread.

In embodiments, the tampon comprises at least two superposed restrictor bands arranged perpendicular to each other before folding.

In embodiments, the restrictor band is designed to have a length that ensures the restrictor band does not disturb the natural development of the geometry of the tampon body when the tampon is placed in contact with a fluid that is to be absorbed.

The two variants of the invention can be combined in one sanitary tampon. In such combined sanitary tampon in a preferred embodiment it is provided that the zone defined by the solid hydrophobic substance and the zone defined by the at least one restriction member are located in a proximal area of the body of the tampon.

The disclosure is also directed to a method for producing a tampon, wherein the method finally comprises, after a step of folding and/or of compression and/or of impregnation of an absorbent material such as an absorbent strip including a hydrophobic substance, a step of heating to a temperature above the melting point of the hydrophobic substance, making it possible to bind the impregnated zones.

The disclosure is also directed to a tampon comprising a saturation indicator at its proximal end. In embodiments, the indicator is formed by the proximal zone situated at the proximal end of the tampon comprising at least one zone in which the expansion and/or the absorption are limited, the zone being formed by partial or total impregnation of the absorbent material of the tampon with a solid hydrophobic substance whose melting point is greater than 37 degrees centigrade In embodiments, the indicator is formed by the proximal zone situated at the proximal end of the tampon comprising at least one zone in which the expansion and/or the absorption are limited, the zone being formed by placement of a restriction member, for example in the form of a ring, string, collar or restrictor band(s) encircling the tampon and allowing it to be maintained in its initial shape in the zone in question, when the tampon comes to inflate under the effect of the absorption. The various embodiments may be separate or in combination with one or more of the other embodiments.

Embodiments will be better understood from the detailed description given below and by reference to the attached drawings, in which:

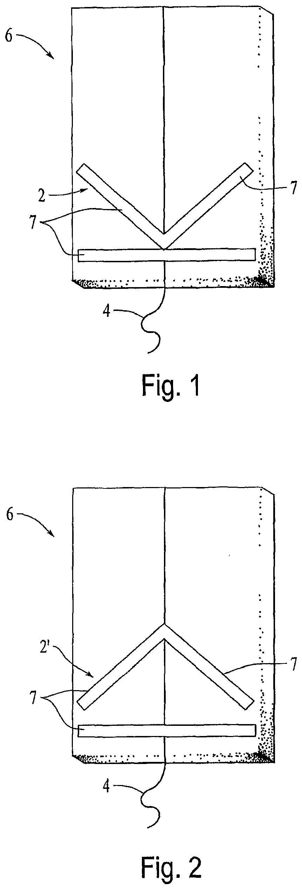

Figures 1 and 2 are diagrammatic views of two strips of absorbent material;

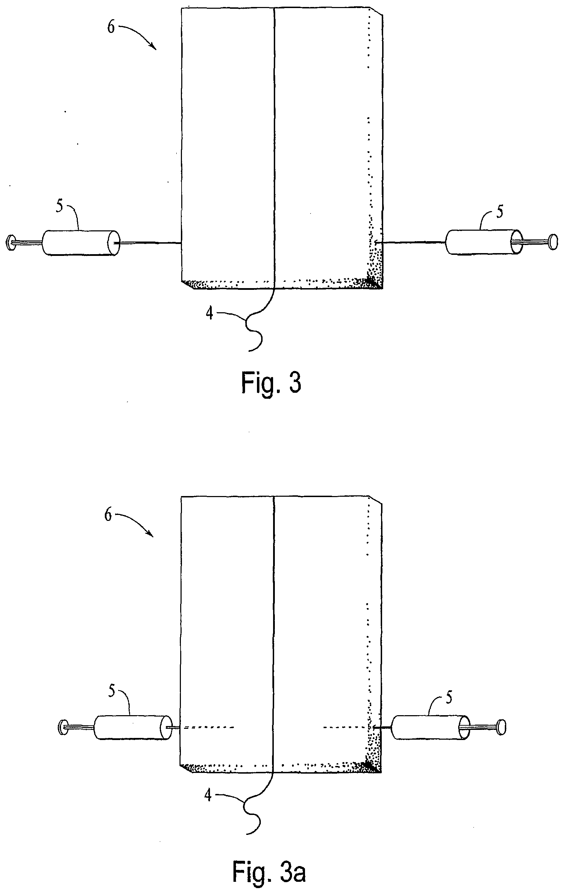

Figures 3 and 3a are diagrammatic views of two other strips of absorbent material during the injection of the hydrophobic substance;

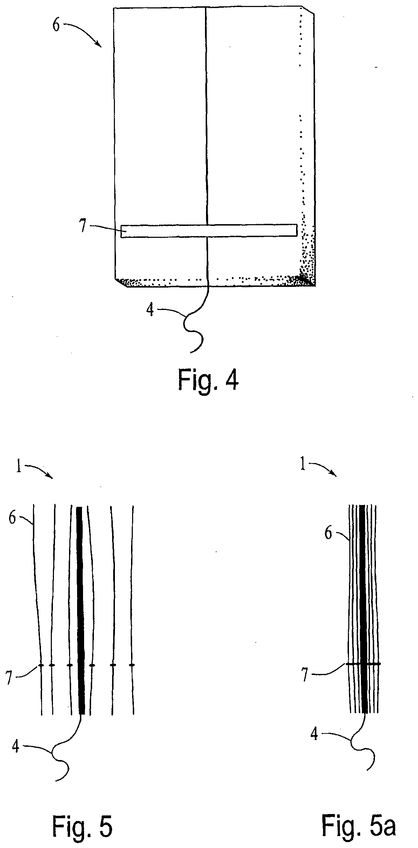

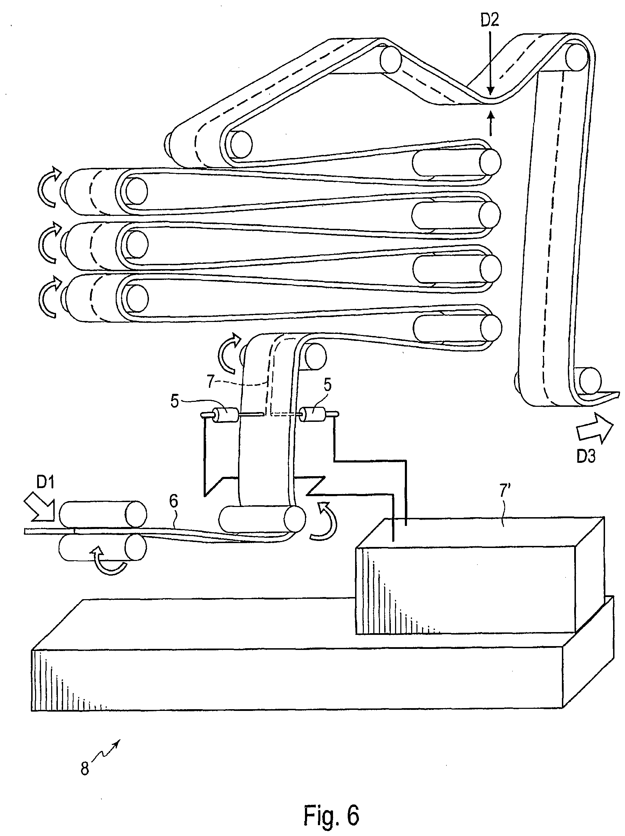

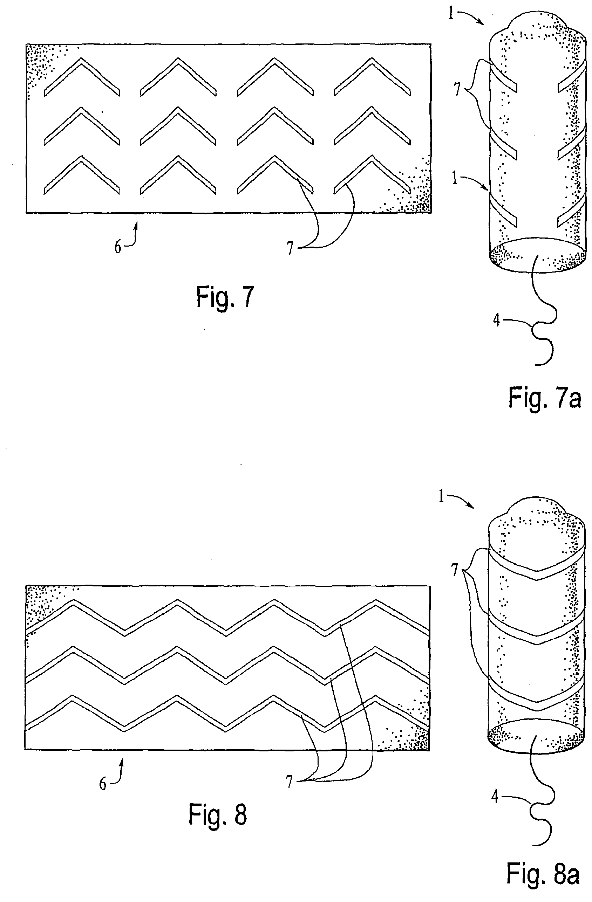

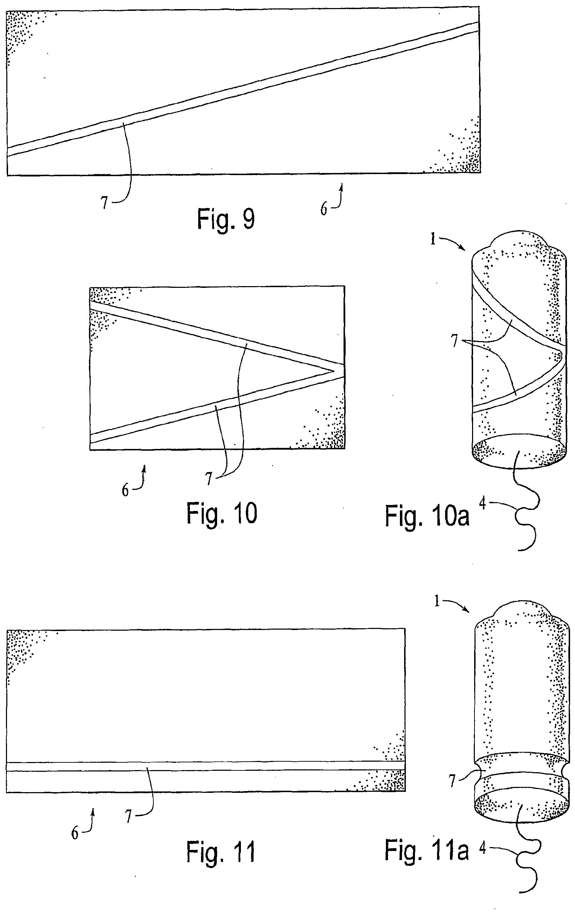

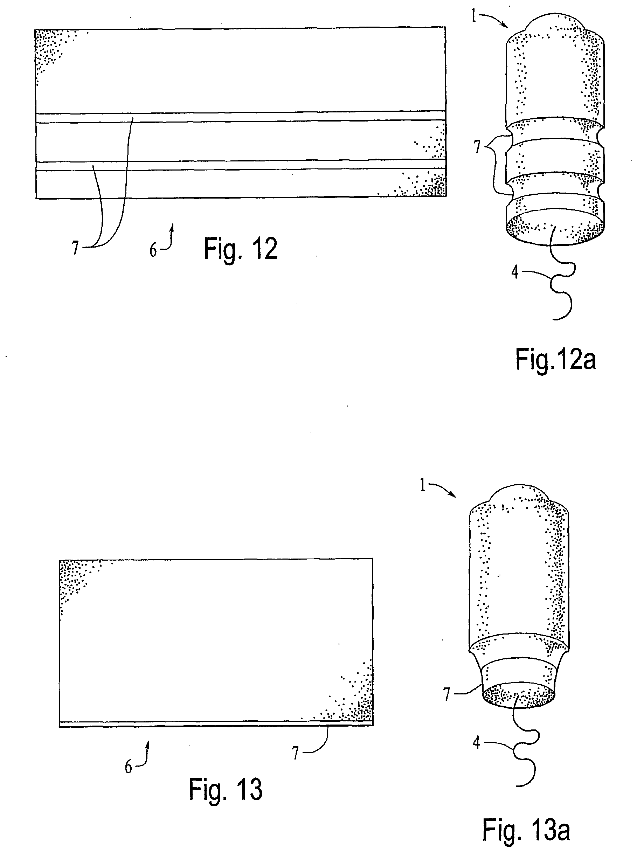

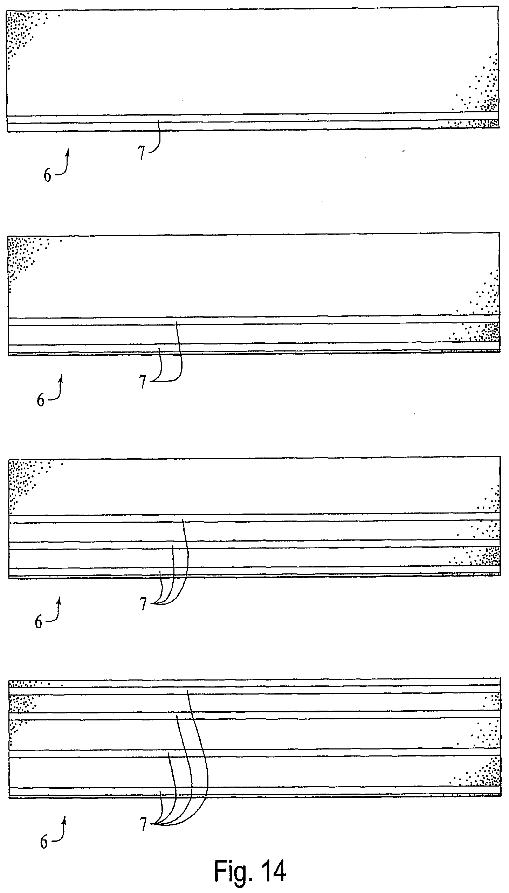

Figure 4 is a diagrammatic view of another strip of absorbent material when laid out flat;

A strip of absorbent material 6 of absorbent material may comprise zones in which the absorbent material is partially or totally impregnated with a solid hydrophobic substance 7 whose melting point is greater than 37 degrees centigrade For example, the hydrophobic substances described herein may have a melting point equal to or greater than 38 degrees centigrade, 39 degrees centigrade, 40 degrees centigrade, 41 degrees centigrade, 42 degrees centigrade, 43 degrees centigrade, 44 degrees centigrade, 45 degrees centigrade, or 46 degrees centigrade In embodiments, the hydrophobic substance has a melting point equal to or greater than 47 degrees centigrade, 49 degrees centigrade, 51 degrees centigrade, 53 degrees centigrade, 55 degrees centigrade, 57 degrees centigrade, 59 degrees centigrade, 61 degrees centigrade, or 63 degrees centigrade In embodiments, the hydrophobic substance has a melting point equal to or greater than 68 degrees centigrade, 69 degrees centigrade, 70 degrees centigrade, 71 degrees centigrade, 72 degrees centigrade, 73 degrees centigrade, 74 degrees centigrade, 75 degrees centigrade, or 76 degrees centigrade

The hydrophobic substances preferably do not dissolve in bodily fluids and preferably have a melting point that is higher than the internal temperature of the human body (37 degrees C) so that the hydrophobic substance remains solid while the tampon is in a vagina. Preferably, the hydrophobic substance has a melting point that is 45 degrees centigrade or higher so that the hydrophobic substance remains solid during shipping and/or storage of the tampons where the tampons may be exposed to temperatures greater or significantly greater than 37 degrees centigrade However, it is preferred that the melting point of the hydrophobic substance does not exceed 120 degrees centigrade, and more preferably does not exceed 100 degrees centigrade, thus making it more economical to manufacture the tampons in terms of costs and time. The lower the melting temperature of the hydrophobic substance, the less energy and time required to melt the hydrophobic substance for impregnation of the absorbent material and to allow or cause the impregnated hydrophobic substance to solidify.

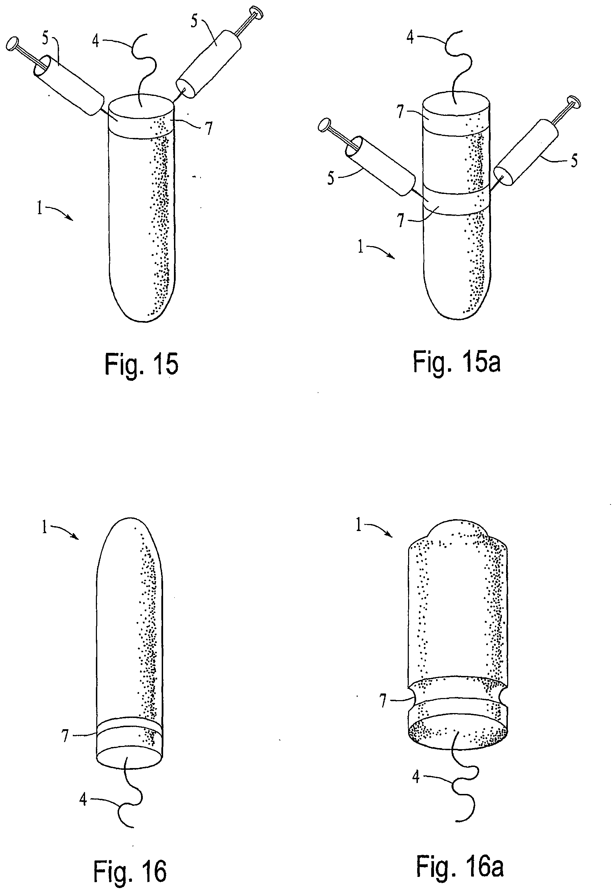

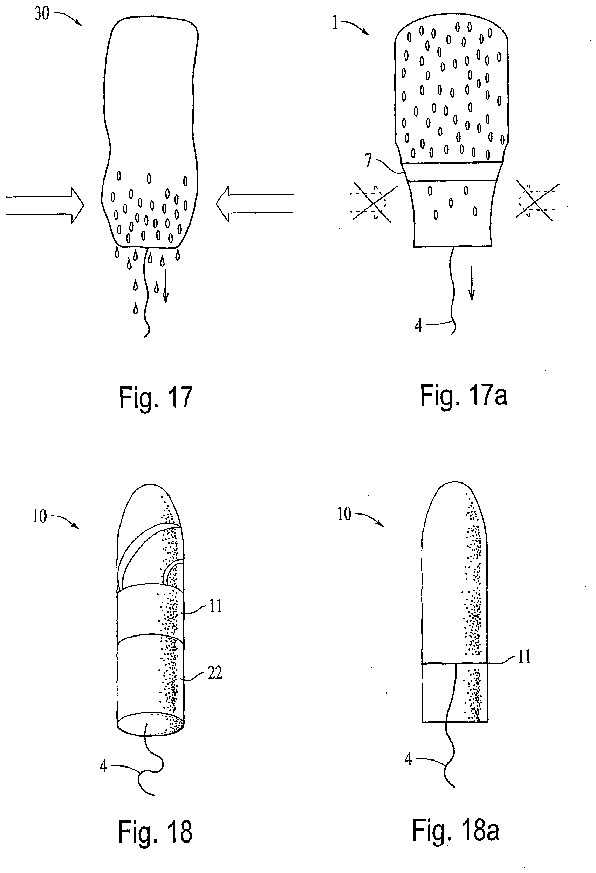

The mode of functioning of the absorption of the tampon 1 is illustrated in Figures 16, 16a and 17a. The absorption is improved by the barrier effect provided by the impregnation with the hydrophobic substance 7. This therefore makes it possible to reduce the first leaks. The zones that are impregnated are nondeformable or are minimally deformable, and they are not expanded, or are only minimally expanded, by the absorption of the flow of fluid. Moreover, such a tampon 1 may be designed to have an improved fluid absorption capacity of up to about 60 percent.

As is shown more specifically in Figure 17, a traditional tampon 30 will tend to allow an untimely quantity of blood to escape via the proximal end and the withdrawal thread, on account of pressure exerted on the tampon by the muscles near the vaginal opening during removal of the tampon 30. This pressure is represented schematically by the white arrows in Figure 17. By contrast, a tampon 1 configured with a fluidic barrier formed by a hydrophobic substance 7 as disclosed herein and as shown in Figure 17a, counteracts this phenomenon by reducing the amount of blood that flows into the proximal area of the tampon during withdrawal of the tampon.

When the impregnation is partial and/or the tampon 1 does not undergo heating after formation of the tampon 1 with preimpregnated strip of absorbent material 6, and/or after the impregnation, the barrier formed simply has a filtering effect and makes it possible to increase the absorption by increasing the contact time between the flow and the absorbent material from which the tampon 1 is made.

According to a second embodiment of a tampon 10, the means for limiting the expansion and/or the absorption are formed by at least one restriction member 11 encircling the corresponding zone of the body and allowing the zone to be maintained in its initial shape when the body comes to inflate under the effect of the absorption. This restriction member 11 may, for example, be in the form of a ring, a string or a collar encircling the body of the tampon 10, as is illustrated in Figure 18.

In an embodiment, the zone that is compressed or whose expansion and/or absorption are limited, functions as a means of reducing the passage of the fluid.

In another embodiment, the restriction member 11 may be formed with a string, where at least a portion of the string is wrapped, tied, fastened, or otherwise affixed around the body of the tampon. As shown schematically in Figure 18a, a withdrawal thread 4 may also be connected to and/or function as the restriction member 11.

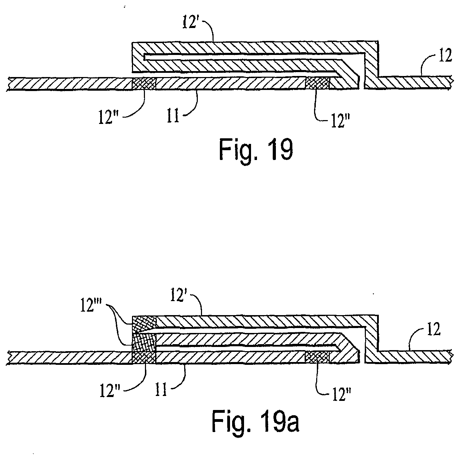

In a first version of the second variant, the ring forming the restriction member 11 is be formed with the protector 12 or packaging of the tampon 10, in the form of a wrapper, which is cut or pre-slit or breakable so that the ring is separable from the protector 12 or packaging of the tampon 10, as is illustrated in Figures 19 and 19a.

This protector 12 has a tab 12' for guaranteeing the protection of the ring 11 intended to remain in place after the insertion. During use of the tampon 10, the user takes hold of this tab 12' in order to fragment the protector 12 at the area of two weld points or bonding points 12". The ring 11 will then be formed by the remaining part of the protector 12 around the tampon 10.

Moreover, and as is shown in Figure 19a, the tab 12' may be reattached to the protector 12 via weld points or bonding points 12"'. This variant is particularly advantageous from the point of view of hygiene, because the ring 11 is free of any prior contact with the exterior before detachment of the protector 12.

The position of the ring 11 may be centered with respect to the distal and/or proximal ends and/or offset toward one of the ends.

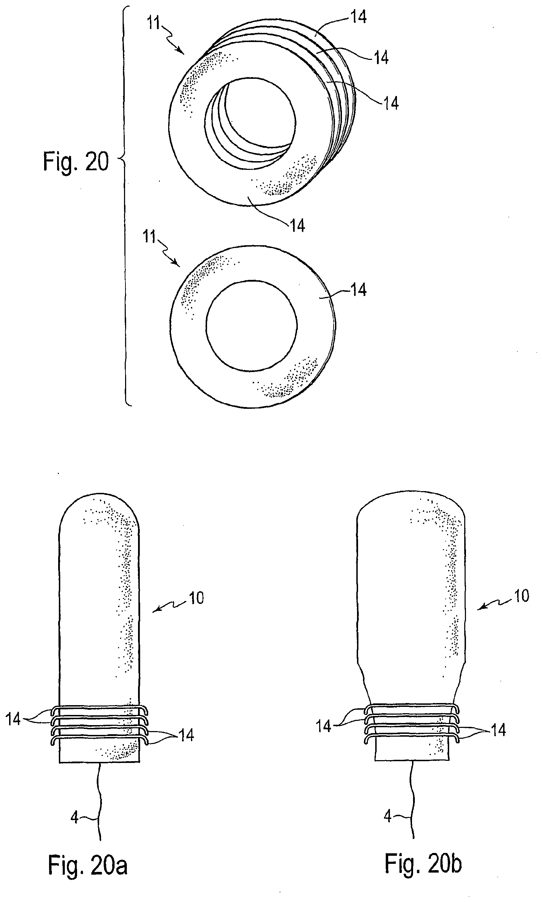

As is shown in Figures 20 and 20a, the ring may be formed by a single washer 14 or by a plurality of stacked washers 14, such as 2, 3, 4, 5, 6, 7, 8, 9, or 10 or more washers. The orifice of each washer 14 has a diameter substantially equal to that of the compressed body of the tampon 10. Figures 20a and 20b show a ring having four stacked washers 14.

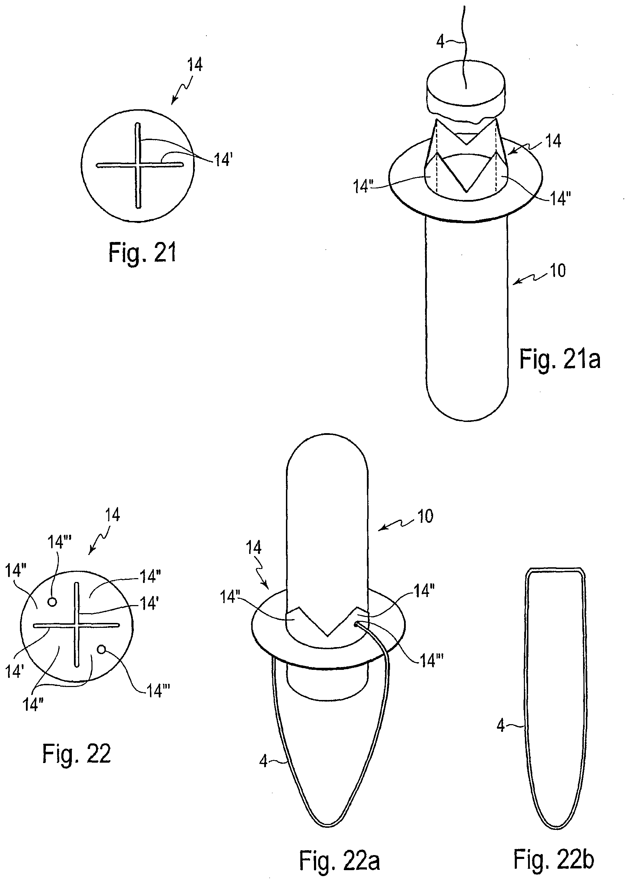

As is shown in Figure 21, the orifice of each washer 14 is preferably formed by making two slits 14' perpendicular to each other, the effect of which is to delimit four substantially triangular flaps 14" which are oriented upward or downward depending on whether the washer 14 is introduced via the top or bottom of the tampon 10. However, the orifice may be formed by two slits that are not perpendicular to each other, or by more than two slits. In Figure 21a, the washer 14 has been slid on from the bottom of the tampon 10. The washers 14 may be secured to the tampon by affixing one or more of the triangular flaps 14" to the tampon by hot pressing, hot melting, sewing, or any other method, as schematically depicted by the dashed lines in Figure 21a.

Moreover, as is shown in Figure 22, two opposite flaps 14" may each comprise a hole 14"' in such a way as to allow the passage of the withdrawal thread 4 shown in Figure 22b. In Figure 22a, the washer 14 thus formed has been slid on from the top of the tampon 10.

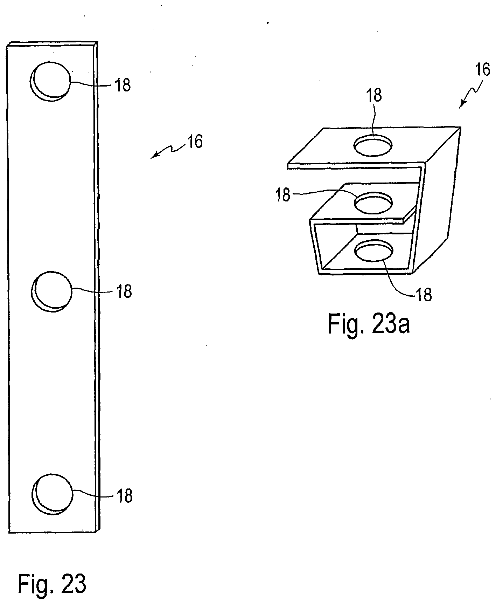

Referring to Figures 23 to 31, it is noted that each restriction member may be made of a permeable material in the form of a restrictor band 16 or in the form of a cross 17 prior to folding.

More precisely, a restrictor band 16 for the upper part of the tampon 10 has at least one nondeformable or minimally deformable orifice 18 near each of its two ends, and the diameter of this orifice 18 is chosen such as to be equal to that of the body of the compressed tampon 10. Thus, once the tampon 10 is in place, the body of the tampon will inflate under the effect of the absorption, while the zone of the body of the tampon encircled by the edge of the orifices 18 will remain compressed. These orifices 18 may therefore actually play the role of a restriction member, prohibiting the tampon 10 from changing diameter in the desired zones.

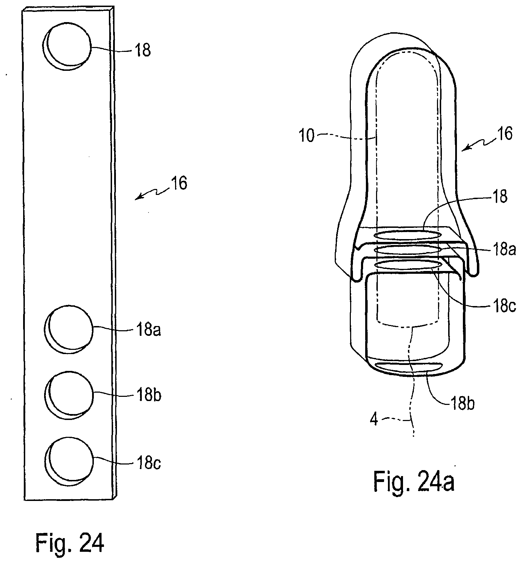

As is shown more specifically in Figures 23 and 24, the restrictor band 16 has a supplementary central orifice 18 when it is a restrictor band intended for the lower part of the tampon 10 and requiring passage of the withdrawal thread 4.

It must also be clearly understood that the same restrictor band 16 may be used for the lower part and the upper part of the tampon 10, as is shown in Figures 24 and 24a. The restrictor band 16 in this case comprise four orifices 18, 18a, 18b, and 18c.

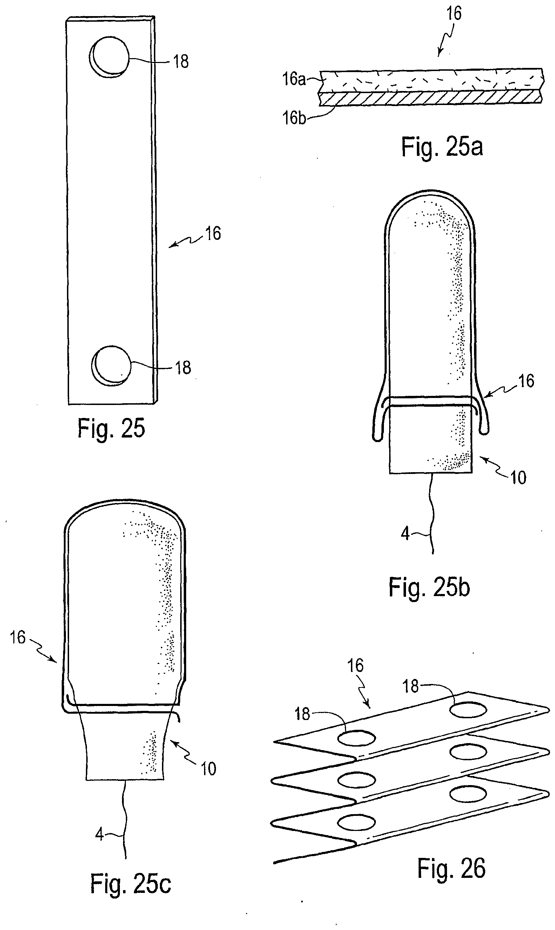

By contrast, the tampon 10 shown in Figure 25b comprises a restrictor band 16, illustrated in Figure 25, that has been folded and then inserted exclusively via the upper part of the tampon 10. Figure 25c shows the tampon 10 after use.

Another restrictor band 16 according to embodiments, as shown in Figure 25a, differs from the one described above in that it comprises, on the one hand, an inner absorbent layer 16a and, on the other hand, an outer impermeable layer 16b connected to the inner layer 16a.

As is shown in Figure 26, the restrictor band 16 may be formed by folding in an accordion configuration.

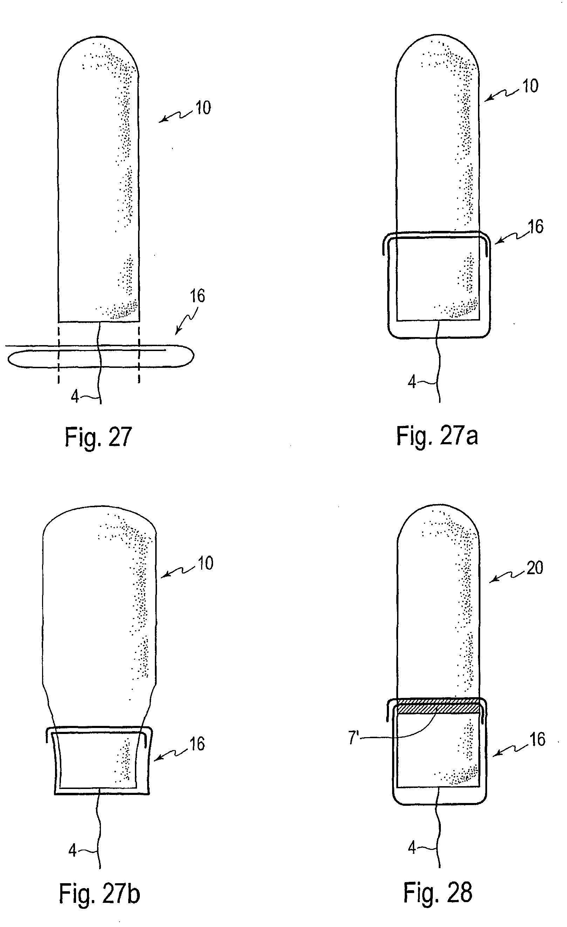

As is shown in Figure 27, the restrictor band 16 is folded in such a way as to align the orifices 18, with a view to then being positioned around the tampon 10, either via the top or via the bottom of the tampon 10, by inserting the body of the tampon 10 through the orifices 18. In Figure 27a, this restrictor band 16 is thus introduced via the bottom of the tampon 10.

Advantageously, and as is shown in particular in Figure 27a, the restrictor band 16 is preferably long enough to leave enough "slack" such that the tampon 10 can dilate. After use, and as is shown in Figure 27b, the restrictor band 16 will enclose the tampon 10 at the bottom part. In this embodiment, reduced or no friction occurs in the area of the vaginal wall during spasms. Indeed, the slight movement of translation of the tampon due to each spasm does not cause any movement of the restrictor band 16 itself because of the "slack," which allows the restrictor band 16 to remain motionless in relation to the vaginal wall. Furthermore, withdrawal of the tampon is easier because it happens schematically in two steps due to the "slack." In other words, the "slack" in the restrictor band 16 allows some freedom of movement of the body of the tampon with respect to the restrictor band 16. Thus, when withdrawing the device, the body of the tampon will move first until it has been moved a sufficient distance that the "slack" in the restrictor band 16 is taken up, whereby the movement of the body of the tampon exerts enough tension on the restrictor band 16 that the body of the tampon and the restrictor band 16 move relative to the walls of the vagina at an equal rate. Also, the pulling force on the body of the tampon elongates the tampon in the axial direction, which decreases the radial diameter of the body of the tampon. As a result, the body of the tampon exerts less radial force on the restrictor band 16, which reduces the frictional force between the walls of the vagina and the restrictor band 16. Accordingly, the longitudinal forces that must be applied to the tampon to withdraw the device are lower than those required with a traditional tampon.

For still greater efficacy, it must be clearly understood that the limitation of the expansion and/or of the absorption of one or more zones of a tampon 20 according to the invention is able to be obtained by simultaneous use, as shown in Figure 28, of a fluidic barrier 7', formed with the aid of a hydrophobic substance 7, and also of a restriction member, such as a restrictor band 16. The fluidic barrier 7' and the zone formed by a restriction member may be located in a proximate area of the tampon, and may be located at substantially the same distance from the proximal end of the tampon.

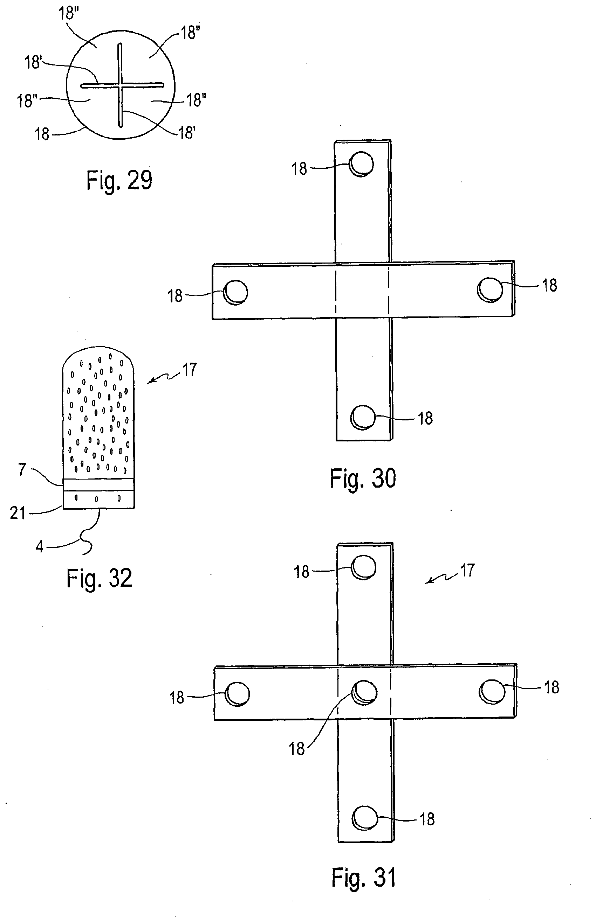

As is shown in Figure 29, each orifice 18 of the restrictor band 16 may be formed by making two slits 18' perpendicular to each other, the effect of which is to delimit four substantially triangular flaps 18" which is oriented upward or downward depending on whether the orifice 18 is introduced via the top or via the bottom of the tampon 10. However, the orifice may be formed by two slits that are not perpendicular to each other, or by more than two slits. A restrictor band 16 may be secured to the tampon by affixing one or more of the triangular flaps 18" to the tampon by hot pressing, hot melting, sewing, or any other method.

Of course, the material used to form the restrictor band 16 is chosen to be able to resist the pressure of the tampon 10, 20 in the area of the orifices 18.

In the case where the restriction member has the form of a cross 17, each of the four ends comprises an orifice 18, as is illustrated in Figure 30. If it is a restriction member for the lower part of the tampon 10, a supplementary central orifice 18 is formed for passage of the withdrawal thread 4, as shown in Figure 31.

It must also be clearly understood that this cross 17 may alternatively be produced with the aid of two separate restrictor bands 16 that are superposed in the area of their central parts and are arranged perpendicular to each other. In the case where this cross 17 is arranged in the lower part of the tampon, the central orifices 18 of the two restrictor bands 16 are superposed in such a way as to permit passage of the withdrawal thread 4.

The restriction member, whether in the form of a restrictor band 16 or a cross 17, is designed to provide some "slack," such that it is able to adapt to the geometry of the body once it has developed in shape under the effect of the inflation associated with the absorption of the fluid, as is illustrated in particular in Figures 24 and 24a. For this reason, the tampon 10 is able to deploy fully in the upper part without being impeded by the restrictor band 16 or cross 17 arranged around it.

Generally speaking, increasing the number of orifices 18 makes it possible to reduce the stresses that have to be withstood by each orifice 18.

In embodiments, the tampon comprises a saturation indicator at its proximal end. A saturation indicator is understood as a means allowing the user to verify the state of saturation or nonsaturation of the tampon by simple contact.

As is illustrated in Figure 32, the proximal zone 21 situated at the proximal end of the tampon 1 comprises at least one zone in which the expansion and/or the absorption are limited, this zone being formed by partial or total impregnation of the absorbent material of the tampon 1 with a solid hydrophobic substance 7 whose melting point is greater than 37 degrees centigrade This proximal zone 21 may then serve as a saturation indicator zone.

In fact, because of the presence of the zone in which the expansion and/or the absorption are limited, the proximal zone 21 is soaked only when the tampon 1 is saturated. This proximal zone 21 may thus be used as a saturation indicator. If the proximal zone 21 is neither soaked nor inflated then this indicates to the user that the tampon 1 is not saturated.

In embodiments, the tampon 10 illustrated in Figure 17 is notable in that the saturation indicator is formed by the proximal zone 22 situated at the proximal end of the tampon 10 comprising at least one zone in which the expansion and/or the absorption are limited by placement of at least one restriction member 11, for example in the form of a ring, string, collar or one or more restrictor bands encircling the zone of the body of the tampon and allowing it to be maintained at this location in its initial shape when the body of the tampon inflates under the effect of the absorption.

Although the invention has been described in connection with particular illustrative embodiments, it will be clear that it is not in any way limited to these embodiments and that it covers all the technical equivalents of the means described, and their combinations, insofar as these come within the scope of the invention.

## Claims

1. Sanitary tampon, comprising a body (1) of absorbent material (6), a withdrawal thread (4) fixed to the absorbent material (6), and an area of a solid hydrophobic substance (7) that has a melting point greater than 37 °C, wherein the body (1) has a distal end and an opposite proximal end, the withdrawal thread (4) extending from the proximal end of the body (1), **characterized in that** the solid hydrophobic substance (7) is provided in at least one zone of the absorbent material (6) and at least partially impregnates the absorbent material (6) of the body (1), the at least one zone containing the solid hydrophobic substance (7) is situated at the proximal end of the body (1), so that the solid hydrophobic substance (7) in the at least one zone forms a fluidic barrier that limits absorption and expansion by the zone with the solid hydrophobic substance (7) when the body (1) contacts an absorbable fluid, said hydrophobic substance (7) is stearin.

2. Tampon according to claim 1, **characterized in that** the withdrawal thread (4) is at least partially impregnated with a solid hydrophobic substance having a melting point that is greater than 37 °C.

3. Tampon according to claim 1 or 2, **characterized in that** the hydrophobic substance (7) extends transversely across substantially an entire cross-section of the tampon.

4. Tampon according to any one of the preceding claims, **characterized in that** the at least one zone containing the solid hydrophobic substance (7) is situated at a distance of more than 0,5 mm from the proximal end of the body (1).

5. Tampon according to any one of the preceding claims, **characterized in that** the at least one zone containing the solid hydrophobic substance (7) forms a spiral inside and outside the tampon.

6. Tampon according to any one of the preceding claims, **characterized in that** the body (1) comprises another zone containing a slid hydrophobic substance (7), situated about halfway between the two ends of the body (1).

7. Tampon according to any one of the preceding claims, **characterized in that** the body (1) comprises an impregnation zone (7) in the form of a funnel configured to channel flow toward the proximal end of the body (1).

8. Tampon according to any one of the claims 1 to 7, **characterized in that** the body (1) further comprises an impregnation zone in the form of an upturned funnel configured to divert flow from a center of the body (1).

9. Tampon according to any one of the preceding claims, **characterized in that** the solid hydrophobic substance (7) has a melting point that is in a range of 45 °C to 120 °C, preferably in a range of 68 °C to 10 °C.

10. Sanitary tampon, comprising a body (1) of absorbent material (6) and a withdrawal thread (4) fixed to the absorbent material (6), wherein the body (1) has a distal end and an opposite proximal end, the withdrawal thread (4) extending from the proximal end of the body (1), and wherein there is a zone of the body (1) in which expansion and/or absorption are limited, **characterized in that** the zone in which expansion and/or absorption is limited is defined by at least one restriction member (11) encircling a portion of the body (1) of the tampon, wherein the restriction member (11) is configured to permanently restrict a diameter of the body (1) of the tampon at said zone from expanding upon absorption of a fluid by the tampon, wherein the restriction member (11) comprises at least one ring, washer, collar, or foldable restrictor band, and wherein the restriction member (11) comprises a ring that comprises a separable portion of a wrapper (12) on the body (1).

11. Tampon according to claim 10, **characterized in that** an outer surface of the restriction member (11) comprises at least one smooth zone configured to reduce friction between the tampon and a vagina during removal of the tampon.

12. Sanitary tampon, comprising a body (1) of absorbent material (6) and a withdrawal thread (4) fixed to the absorbent material (6), wherein the body (1) has a distal end and an opposite proximal end, the withdrawal thread (4) extending from the proximal end of the body (1), and wherein there is a zone of the body (1) in which expansion and/or absorption are limited, **characterized in that** the zone in which expansion and/or absorption is limited is defined by at least one restriction member (11) encircling a portion of the body (1) of the tampon, wherein the restriction member (11) is configured to permanently restrict a diameter of the body (1) of the tampon at said zone from expanding upon absorption of a fluid by the tampon, wherein the restriction member (11) comprises at least one ring, washer, collar, or foldable restrictor band, and wherein the restriction member (11) comprises at least one restrictor band (16) having two ends, and said restrictor band (16) comprises at least one orifice (18) near each of the two ends of the restrictor band (16), the at least one said orifice (18) having a diameter that is substantival equal to a diameter of the tampon when the tampon is compressed.

13. Tampon according to claim 12, **characterized in that** an outer surface of the restriction member (11) comprises at least one smooth zone configured to reduce friction between the tampon and a vagina during removal of the tampon.

14. Tampon according to claim 12 or 13, **characterized in that** the restrictor band (16) further comprises at least a third orifice (18) in a central part of the restrictor band (16) for passage of a withdrawal thread (4).

15. Tampon according to claim 12 or 13, **characterized in that** at least one said orifice (18) in the restrictor band (16) is defined by a plurality of slits.

16. Tampon according to claim 15, **characterized in that** at least one said orifice (18) in the restrictor band (16) is defined by two slits perpendicular to each other, in such a way as to delimit four flaps.

17. Tampon according to claim 15, **characterized in that** at least one of the four flaps is affixed to the body (1) of the tampon.

18. Tampon according to any one of the claims 10 to 12, **characterized in that** the tampon comprises at least two superposed restrictor bands (16) arranged perpendicular to each other at a location at which the restrictor bands (16) are superposed.

19. Tampon according to any one of the claims 10 to 12, **characterized in that** the restriction member (11) comprises at least one restrictor band (16). and the restrictor band (16) is configured to have a length that permits expansion of the body (1) outside said zone when the body (1) is placed in contact with a fluid that is to be absorbed.

20. Sanitary tampon, comprising a body (1) of absorbent material (6) and a withdrawal thread (4) fixed to the absorbent material (6), wherein the body (1) has a distal end and an opposite proximal end, the withdrawal thread (4) extending from the proximal end of the body (1), and wherein there is a zone of the body (1) in which expansion and/or absorption are limited, **characterized in that** the zone in which expansion and/or absorption is limited is defined by at least one restriction member (11) encircling a portion of the body (1) of the tampon, wherein the restriction member (11) is configured to permanently restrict a diameter of the body (1) of the tampon at said zone from expanding upon absorption of a fluid by the tampon, wherein the restriction member (11) comprises at least one ring, washer, collar, or foldable restrictor band, and wherein the restriction member (11) comprises at least one washer having an orifice that is defined by a plurality of slits.

21. Tampon according to claim 20, **characterized in that** an outer surface of the restriction member (11) comprises at least one smooth zone configured to reduce friction between the tampon and a vagina during removal of the tampon.

22. Tampon according to claim 20, **characterized in that** the restriction member (11) comprises at least one washer having an orifice that is defined by two slits perpendicular to each other in such a way as to delimit four flaps.

23. Tampon according to claim 22, **characterized in that** at least one of the four flaps is affixed to the body (1) of the tampon.

24. Tampon according to claim 22 or 23, **characterized in that** two opposite flaps each comprise a hole permitting passage of a withdrawal thread.

25. Tampon according to any one of the claims 10 to 24, **characterized in that** the restriction member (11) comprises the withdrawal thread (4) that is attached to the restriction member (11).

26. Tampon according to any one of the claims 1 to 9 and also to any one of the claims 10 to 25, **characterized in that** the zone defined by the solid hydrophobic substance (7) and the zone defined by the at least one restriction member (11) are located in a proximal area of the body (1) of the tampon.

27. Tampon according to claim 26, **characterized in that** the zone defined by the solid hydrophobic substance (7) and the zone defined by the at least one restriction member (11) are located at substantially the same distance from a proximal end of the body (1) of the tampon.

28. A method for manufacturing a sanitary tampon, comprising: injecting a solid hydrophobic substance having a melting point that is greater than 37 °C into a thickness of a strip of absorbent material, and forming said strip of absorbent material into a body of a tampon.

29. Method according to claim 28, **characterized in that** the hydrophobic substance is injected discontinuously.

30. Method according to claim 28 to 29, **characterized in that** it further comprises a step of folding and optionally of compressing the strip of absorbent material, followed by a step of heating said strip of absorbent material to a temperature above a melting point of the hydrophobic substance, thereby binding together two or more portions of said strip containing said hydrophobic substance.

31. Method according to any one of the claims 28 to 30, **characterized in that** the hydrophobic substance is selected from the group consisting of hydrogenated and nonhydrogenated oils and fats.

32. Method according to any one of the claims 28 to 30, **characterized in that** the hydrophobic substance is selected from the group consisting of natural and synthetic waxes, wherein, preferably, the hydrophobic substance is beeswax or carnauba wax.

33. Method according to any one of the claims 28 to 30, **characterized in that** the hydrophobic substance is hydrogenated petrolatum.

## Patentansprüche

1. Sanitärtampon, umfassend einen Körper (1) aus saugfähigem Material (6), einen Rückholfaden (4), der an dem saugfähigen Material (6) befestigt ist, und einen Bereich aus einer festen hydrophoben Substanz (7) mit einem Schmelzpunkt über 37 °C, wobei der Körper (1) ein distales Ende und ein gegenüberliegendes proximales Ende aufweist, wobei sich der Rückholfaden (4) von dem proximalen Ende des Körpers (1) aus erstreckt, **dadurch gekennzeichnet, dass** die feste hydrophobe Substanz (7) in mindestens einer Zone des saugfähigen Materials (6) bereitgestellt ist und das saugfähige Material (6) des Körpers (1) mindestens teilweise imprägniert, wobei die mindestens eine Zone, welche die feste hydrophobe Substanz (7) enthält, an dem proximalen Ende des Körpers (1) angeordnet ist, so dass die feste hydrophobe Substanz (7) in der mindestens einen Zone eine Fluidbarriere bildet, die Absorption und Expansion durch die Zone mit der festen hydrophoben Substanz (7) bei Kontakt des Körpers (1) mit einem absorbierbaren Fluid begrenzt, wobei die hydrophobe Substanz (7) Stearin ist.

2. Tampon nach Anspruch 1, **dadurch gekennzeichnet, dass** der Rückholfaden (4) mindestens teilweise mit einer festen hydrophoben Substanz mit einem Schmelzpunkt über 37 °C imprägniert ist.

3. Tampon nach Anspruch 1 oder 2, **dadurch gekennzeichnet, dass** sich die hydrophobe Substanz (7) quer im Wesentlichen über den gesamten Querschnitt des Tampons erstreckt.

4. Tampon nach einem der vorhergehenden Ansprüche, **dadurch gekennzeichnet, dass** die mindestens eine Zone, die die feste hydrophobe Substanz (7) enthält, in einem Abstand von mehr als 0,5 mm von dem proximalen Ende des Körpers (1) angeordnet ist.

5. Tampon nach einem der vorhergehenden Ansprüche, **dadurch gekennzeichnet, dass** die mindestens eine Zone, die die feste hydrophobe Substanz (7) enthält, eine Spirale auf der Innenseite und Außenseite des Tampons bildet.

6. Tampon nach einem der vorhergehenden Ansprüche, **dadurch gekennzeichnet dass** der Körper (1) eine andere Zone umfasst, die eine gleitende hydrophobe Substanz (7) enthält, die ungefähr in der Mitte zwischen den beiden Enden des Körpers (1) angeordnet ist.

7. Tampon nach einem der vorhergehenden Ansprüche, **dadurch gekennzeichnet, dass** der Körper (1) eine Imprägnierungszone (7) in Form eines Trichters umfasst, der darauf ausgelegt ist, Fluss zum proximalen Ende des Körpers (1) zu leiten.

8. Tampon nach einem der Ansprüche 1 bis 7, **dadurch gekennzeichnet, dass** der Körper (1) ferner eine Imprägnierungszone in Form eines nach oben gedrehten Trichters umfasst, der darauf ausgelegt ist, Fluss von einer Mitte des Körpers (1) umzuleiten.

9. Tampon nach einem der vorhergehenden Ansprüche, **dadurch gekennzeichnet, dass** die feste hydrophobe Substanz (7) einen Schmelzpunkt aufweist, der in einem Bereich von 45 °C bis 120 °C liegt, vorzugsweise in einem Bereich von 68 °C bis 10 °C.

10. Sanitärtampon, umfassend einen Körper (1) aus saugfähigem Material (6) und einen Rückholfaden (4), der an dem saugfähigen Material (6) befestigt ist, wobei der Körper (1) ein distales und ein gegenüberliegendes proximales Ende aufweist, wobei sich der Rückholfaden (4) von dem proximalen Ende des Körpers (1) aus erstreckt, und wobei es eine Zone des Körpers (1) gibt, in der Expansion und/oder Absorption begrenzt sind, **dadurch gekennzeichnet, dass** die Zone, in der Expansion und/oder Absorption begrenzt sind, von mindestens einem Begrenzungselement (11) definiert wird, das einen Abschnitt des Körpers (1) des Tampons umgibt, wobei das Begrenzungselement (11) darauf ausgelegt ist, dauerhaft einen Durchmesser des Körpers (1) des Tampons in der Zone in Bezug auf eine Expansion bei Absorption eines Fluids durch den Tampon zu begrenzen, wobei das Begrenzungselement (11) mindestens einen Ring, eine Unterlegscheibe, einen Kragen oder ein faltbares Begrenzungsband umfasst, und wobei das Begrenzungselement (11) einen Ring umfasst, der einen trennbaren Abschnitt einer Umhüllung (12) auf dem Körper (1) umfasst.

11. Tampon nach Anspruch 10, **dadurch gekennzeichnet, dass** eine Außenfläche des Begrenzungselements (11) mindestens eine glatte Zone umfasst, die darauf ausgelegt ist, die Reibung zwischen dem Tampon und einer Vagina während des Entfernens des Tampons zu reduzieren.

12. Sanitärtampon, umfassend einen Körper (1) aus saugfähigem Material (6) und einen Rückholfaden (4), der an dem saugfähigem Material (6) befestigt ist, wobei der Körper (1) ein distales und ein gegenüberliegendes proximales Ende aufweist, wobei sich der Rückholfaden (4) von dem proximalen Ende des Körpers (1) aus erstreckt, und wobei es eine Zone des Körpers (1) gibt, in der Expansion und/oder Absorption begrenzt sind, **dadurch gekennzeichnet, dass** die Zone, in der Expansion und/oder Absorption begrenzt sind, von mindestens einem Begrenzungselement (11) definiert wird, das einen Abschnitt des Körpers (1) des Tampons umgibt, wobei das Begrenzungselement (11) darauf ausgelegt ist, dauerhaft einen Durchmesser des Körpers (1) des Tampons in der Zone in Bezug auf eine Expansion bei Absorption eines Fluids durch den Tampon zu begrenzen, wobei das Begrenzungselement (11) mindestens einen Ring, eine Unterlegscheibe, einen Kragen oder ein faltbares Begrenzungsband umfasst, und wobei das Begrenzungselement (11) mindestens ein Begrenzungsband (16) mit zwei Enden umfasst, und das Begrenzungsband (16) mindestens eine Öffnung (18) neben jedem der beiden Enden des Begrenzungsbands (16) umfasst, wobei die mindestens eine Öffnung (18) einen Durchmesser aufweist, der einem Durchmesser des Tampons im Wesentlichen gleicht, wenn der Tampon zusammengepresst wird.

13. Tampon nach Anspruch 12, **dadurch gekennzeichnet, dass** eine Außenfläche des Begrenzungselements (11) mindestens eine glatte Zone umfasst, die darauf ausgelegt ist, die Reibung zwischen dem Tampon und einer Vagina während des Entfernens des Tampons zu verringern.

14. Tampon nach Anspruch 12 oder 13, **dadurch gekennzeichnet, dass** das Begrenzungsband (16) ferner mindestens eine dritte Öffnung (18) in einem mittleren Teil des Begrenzungsbands (16) zur Durchführung eines Rückholfadens (4) umfasst.

15. Tampon nach Anspruch 12 oder 13, **dadurch gekennzeichnet, dass** mindestens eine der Öffnungen (18) in dem Begrenzungsband (16) von einer Vielzahl von Schlitzen definiert wird.

16. Tampon nach Anspruch 15, **dadurch gekennzeichnet, dass** mindestens eine der Öffnungen (18) in dem Begrenzungsband (16) durch zwei Schlitze senkrecht zueinander definiert wird, so dass vier Lappen begrenzt werden.

17. Tampon nach Anspruch 15, **dadurch gekennzeichnet, dass** mindestens einer der vier Lappen an dem Körper (1) des Tampons befestigt ist.

18. Tampon nach einem der Ansprüche 10 bis 12, **dadurch gekennzeichnet, dass** der Tampon mindestens zwei übereinander angeordnete Begrenzungsbänder (16) umfasst, die senkrecht zueinander an einer Stelle angeordnet sind, an der die Begrenzungsbänder (16) übereinander angeordnet sind.

19. Tampon nach einem der Ansprüche 10 bis 12, **dadurch gekennzeichnet, dass** das Begrenzungselement (11) mindestens ein Begrenzungsband (16) umfasst, und das Begrenzungsband (16) so ausgelegt ist, dass es eine Länge aufweist, die eine Ausdehnung des Körpers (1) außerhalb der Zone ermöglicht, wenn der Körper (1) mit einem zu absorbierenden Fluid in Kontakt gebracht wird.

20. Sanitärtampon, umfassend einen Körper (1) aus saugfähigem Material (6) und einen Rückholfaden (4), der an dem saugfähigem Material (6) befestigt ist, wobei der Körper (1) ein distales und ein gegenüberliegendes proximales Ende aufweist, wobei sich der Rückholfaden (4) von dem proximalen Ende des Körpers (1) aus erstreckt, und wobei es eine Zone des Körpers (1) gibt, in der Expansion und/oder Absorption begrenzt sind, **dadurch gekennzeichnet, dass** die Zone, in der Expansion und/oder Absorption begrenzt sind, von mindestens einem Begrenzungselement (11) definiert wird, das einen Abschnitt des Körpers (1) des Tampons umgibt, wobei das Begrenzungselement (11) darauf ausgelegt ist, dauerhaft einen Durchmesser des Körpers (1) des Tampons in der Zone in Bezug auf eine Expansion bei Absorption eines Fluids durch den Tampon zu begrenzen, wobei das Begrenzungselement (11) mindestens einen Ring, eine Unterlegscheibe, einen Kragen oder ein faltbares Begrenzungsband umfasst, und wobei das Begrenzungselement (11) mindestens eine Unterlegscheibe mit einer Öffnung umfasst, die von einer Vielzahl von Schlitzen definiert wird.

21. Tampon nach Anspruch 20, **dadurch gekennzeichnet, dass** eine Außenfläche des Begrenzungselements (11) mindestens eine glatte Zone umfasst, die ausgelegt ist, die Reibung zwischen dem Tampon und einer Vagina während des Entfernens des Tampons zu verringern.

22. Tampon nach Anspruch 20, **dadurch gekennzeichnet, dass** das Begrenzungselement (11) mindestens eine Unterlegscheibe mit einer Öffnung umfasst, die durch zwei Schlitze senkrecht zueinander definiert wird, so dass vier Lappen begrenzt werden.

23. Tampon nach Anspruch 22, **dadurch gekennzeichnet, dass** mindestens einer der vier Lappen an dem Körper (1) des Tampons befestigt ist.

24. Tampon nach Anspruch 22 oder 23, **dadurch gekennzeichnet, dass** zwei gegenüberliegende Lappen jeweils ein Loch umfassen, das Durchführung eines Rückholfadens gestattet.

25. Tampon nach einem der Ansprüche 10 bis 24, **dadurch gekennzeichnet, dass** das Begrenzungselement (11) den Rückholfaden (4) umfasst, der an dem Begrenzungselement (11) befestigt ist.

26. Tampon nach einem der Ansprüche 1 bis 9 sowie nach einem der Ansprüche 10 bis 25, **dadurch gekennzeichnet, dass** die Zone, die durch die feste hydrophobe Substanz (7) definiert wird, und die Zone, die durch das mindestens eine Begrenzungselement (11) definiert wird, in einem proximalen Bereich des Körpers (1) des Tampons angeordnet sind.

27. Tampon nach Anspruch 26, **dadurch gekennzeichnet, dass** die Zone, die durch die feste hydrophobe Substanz (7) definiert wird, und die Zone, die durch das mindestens eine Begrenzungselement (11) definiert wird, im Wesentlichen im selben Abstand von einem proximalen Ende des Körpers (1) des Tampons angeordnet sind.

28. Verfahren zum Herstellen eines Sanitärtampons, umfassend: Einspritzen einer festen hydrophoben Substanz mit einem Schmelzpunkt über 37 °C in eine Dicke eines Streifens aus saugfähigem Material, und Formen des Streifens aus saugfähigem Material in einen Körper eines Tampons.

29. Verfahren nach Anspruch 28, **dadurch gekennzeichnet, dass** die hydrophobe Substanz diskontinuierlich eingespritzt wird.

30. Verfahren nach Anspruch 28 bis 29, **dadurch gekennzeichnet, dass** es ferner einen Schritt des Faltens und optional des Zusammenpressens des Streifens aus saugfähigem Material, gefolgt von einem Schritt des Erwärmens des Streifens aus saugfähigem Material auf eine Temperatur oberhalb des Schmelzpunkts der hydrophoben Substanz umfasst, wodurch zwei oder mehr Abschnitte des Streifens, der die hydrophobe Substanz enthält, miteinander verbunden werden.

31. Verfahren nach einem der Ansprüche 28 bis 30, **dadurch gekennzeichnet, dass** die hydrophobe Substanz ausgewählt ist aus der Gruppe, bestehend aus gehärteten und nicht-gehärteten Ölen und Fetten.

32. Verfahren nach einem der Ansprüche 28 bis 30, **dadurch gekennzeichnet, dass** die hydrophobe Substanz ausgewählt ist aus der Gruppe, bestehend aus natürlichen und synthetischen Wachsen, wobei die hydrophobe Substanz vorzugsweise Bienenwachs oder Carnaubawachs ist.

33. Verfahren nach einem der Ansprüche 28 bis 30, **dadurch gekennzeichnet, dass** die hydrophobe Substanz gehärtetes Petrolatum ist.

## Revendications

1. Tampon hygiénique, comprenant un corps (1) en matière absorbante (6), un fil de retrait (4) fixé sur la matière absorbante (6), et une zone de substance hydrophobe (7) solide ayant un point de fusion supérieur à 37 °C, dans lequel le corps (1) présente une extrémité distale et une extrémité proximale à l'opposé, le fil de retrait (4) s'étendant depuis l'extrémité proximale du corps (1), **caractérisé en ce que** la substance hydrophobe (7) solide est disposée dans au moins une zone de la matière absorbante (6) et imprègne au moins en partie la matière absorbante (6) du corps (1), l'au moins une zone contenant la substance hydrophobe (7) solide est située au niveau de l'extrémité proximale du corps (1), de sorte que la substance hydrophobe (7) solide dans l'au moins une zone forme une barrière anti-fluides qui limite l'absorption et l'expansion par la zone ayant la substance hydrophobe (7) solide quand le corps (1) est en contact avec un fluide absorbable, ladite substance hydrophobe (7) étant stéarine.

2. Tampon selon la revendication 1, **caractérisé en ce que** le fil de retrait (4) est au moins en partie imprégné d'une substance hydrophobe solide ayant un point de fusion supérieur à 37 °C.

3. Tampon selon la revendication 1 ou 2, **caractérisé en ce que** la substance hydrophobe (7) s'étend transversalement sur essentiellement la totalité d'une section transversale du tampon.

4. Tampon selon l'une quelconque des revendications précédentes, **caractérisé en ce que** l'au moins une zone contenant la substance hydrophobe (7) solide est située à une distance de l'extrémité proximale du corps (1) supérieure à 0,5 mm.

5. Tampon selon l'une quelconque des revendications précédentes, **caractérisé en ce que** l'au moins une zone contenant la substance hydrophobe (7) solide forme une spirale à l'intérieur et à l'extérieur du tampon.

6. Tampon selon l'une quelconque des revendications précédentes, **caractérisé en ce que** le corps (1) comprend une autre zone contenant une substance hydrophobe (7) solide, située à environ mi-distance entre les deux extrémités du corps (1).

7. Tampon selon l'une quelconque des revendications précédentes, **caractérisé en ce que** le corps (1) comprend une zone d'imprégnation (7) ayant la forme d'un entonnoir et configurée pour canaliser le flux vers l'extrémité proximale du corps (1).

8. Tampon selon l'une quelconque des revendications 1 à 7, **caractérisé en ce que** le corps (1) comprend en outre une zone d'imprégnation ayant la forme d'un entonnoir tourné vers le haut et configurée pour détourner le flux d'un centre du corps (1).

9. Tampon selon l'une quelconque des revendications précédentes, **caractérisé en ce que** la substance hydrophobe (7) solide a un point de fusion qui se trouve dans une plage de 45°C à 120°C, de préférence dans une plage de 68°C à 10°C.

10. Tampon hygiénique, comprenant un corps (1) en matière absorbante (6) et un fil de retrait (4) fixé sur la matière absorbante (6), dans lequel le corps (1) présente une extrémité distale et une extrémité proximale à l'opposé, le fil de retrait (4) s'étendant depuis l'extrémité proximale du corps (1), et dans lequel il y a une zone du corps (1) dans laquelle la dilatation et / ou l'absorption sont limitées, **caractérisé en ce que** la zone dans laquelle la dilatation et / ou l'absorption sont limitées est délimitée par au moins un élément de restriction (11) encerclant une partie du corps (1) du tampon, dans lequel l'élément de restriction (11) est configuré pour restreindre en permanence un diamètre du corps (1) du tampon au niveau de ladite zone de se dilater lorsque le tampon absorbe un fluide, dans lequel l'élément de restriction (11) comprend au moins un anneau, une rondelle, un collier ou une bande de restriction pliable, et dans lequel l'élément de restriction (11) comprend un anneau avec une portion séparable d'un emballage (12) sur le corps (1).

11. Tampon selon la revendication 10, **caractérisé en ce qu'**une surface extérieure de l'élément de restriction (11) comprend au moins une zone lisse configurée pour réduire le frottement entre le tampon et le vagin pendant le retrait du tampon.

12. Tampon hygiénique, comprenant un corps (1) en matière absorbante (6) et un fil de retrait (4) fixé sur la matière absorbante (6), dans lequel le corps (1) présente une extrémité distale et une extrémité proximale à l'opposé, le fil de retrait (4) s'étendant depuis l'extrémité proximale du corps (1), et dans lequel il y a une zone du corps (1) dans laquelle la dilatation et / ou l'absorption sont limitées, **caractérisé en ce que** la zone dans laquelle la dilatation et / ou l'absorption sont limitées est délimitée par au moins un élément de restriction (11) encerclant une partie du corps (1) du tampon, dans lequel l'élément de restriction (11) est configuré pour restreindre en permanence un diamètre du corps (1) du tampon au niveau de ladite zone de se dilater lorsque le tampon absorbe un fluide, dans lequel l'élément de restriction (11) comprend au moins un anneau, une rondelle, un collier ou une bande de restriction pliable, et dans lequel l'élément de restriction (11) comprend au moins une bande de restriction (16) comportant deux extrémités, et ladite bande de restriction (16) comprend au moins un orifice (18) près de chacune des deux extrémités de la bande de restriction (16), ledit au moins un orifice (18) ayant un diamètre qui est essentiellement égal à un diamètre du tampon quand le tampon est comprimé.

13. Tampon selon la revendication 12, **caractérisé en ce qu'**une surface extérieure de l'élément de restriction (11) comprend au moins une zone lisse configurée pour réduire le frottement entre le tampon et le vagin pendant le retrait du tampon.

14. Tampon selon la revendication 12 ou 13, **caractérisé en ce que** la bande de restriction (16) comprend en outre au moins un troisième orifice (18) dans une partie centrale de la bande de restriction (16) pour laisser passer un fil de retrait (4).

15. Tampon selon la revendication 12 ou 13, **caractérisé en ce que** ledit au moins un orifice (18) dans la bande de restriction (16) est délimité par une pluralité de fentes.

16. Tampon selon la revendication 15, **caractérisé en ce que** ledit au moins un orifice (18) dans la bande de restriction (16) est délimité par deux fentes perpendiculaires l'une à l'autre, de façon à délimiter quatre rabats.

17. Tampon selon la revendication 15, **caractérisé en ce qu'**au moins l'un des quatre rabats est fixé au corps (1) du tampon.

18. Tampon selon l'une quelconque des revendications 10 à 12, **caractérisé en ce que** le tampon comprend au moins deux bandes de restriction (16) superposées et disposées perpendiculairement l'une à l'autre à un endroit auquel les bandes de restriction (16) se superposent.

19. Tampon selon l'une quelconque des revendications 10 à 12, **caractérisé en ce que** l'élément de restriction (11) comprend au moins une bande de restriction (16), et la bande de restriction (16) est configurée pour avoir une longueur qui permet la dilatation du corps (1) à l'extérieur de ladite zone quand le corps (1) est mis en contact avec un fluide qui doit être absorbé.

20. Tampon hygiénique, comprenant un corps (1) en matière absorbante (6) et un fil de retrait (4) fixé sur la matière absorbante (6), dans lequel le corps (1) présente une extrémité distale et une extrémité proximale à l'opposé, le fil de retrait (4) s'étendant depuis l'extrémité proximale du corps (1), et dans lequel il y a une zone du corps (1) dans laquelle la dilatation et / ou l'absorption sont limitées, **caractérisé en ce que** la zone dans laquelle la dilatation et / ou l'absorption sont limitées est délimitée par au moins un élément de restriction (11) encerclant une partie du corps (1) du tampon, dans lequel l'élément de restriction (11) est configuré pour restreindre en permanence un diamètre du corps (1) du tampon au niveau de ladite zone de se dilater lorsque le tampon absorbe un fluide, dans lequel l'élément de restriction (11) comprend au moins un anneau, une rondelle, un collier ou une bande de restriction pliable, et dans lequel l'élément de restriction (11) comprend au moins une rondelle avec un orifice qui est défini par une pluralité de fentes.

21. Tampon selon la revendication 20, **caractérisé en ce qu'**une surface extérieure de l'élément de restriction (11) comprend au moins une zone lisse configurée pour réduire le frottement entre le tampon et le vagin pendant le retrait du tampon.

22. Tampon selon la revendication 20, **caractérisé en ce que** l'élément de restriction (11) comprend au moins une rondelle avec un orifice qui est délimitée par deux fentes perpendiculaires l'une à l'autre, de façon à délimiter quatre rabats.

23. Tampon selon la revendication 22, **caractérisé en ce qu'**au moins l'un des quatre rabats est fixé au corps (1) du tampon.

24. Tampon selon la revendication 22 ou 23, **caractérisé en ce que** deux rabats opposés comprennent chacun un trou permettant le passage d'un fil de retrait.

25. Tampon selon l'une quelconque des revendications 10 à 24, **caractérisé en ce que** l'élément de restriction (11) comprend le fil de retrait (4) qui est fixé à l'élément de restriction (11).

26. Tampon selon l'une quelconque des revendications 1 à 9 et également à l'une quelconque des revendications 10 à 25, **caractérisé en ce que** la zone délimitée par la substance hydrophobe (7) solide et la zone délimitée par l'au moins un élément de restriction (11) sont situées dans une zone proximale du corps (1) du tampon.

27. Tampon selon la revendication 26, **caractérisé en ce que** la zone délimitée par la substance hydrophobe (7) solide et la zone délimitée par l'au moins un élément de restriction (11) sont situées à essentiellement la même distance de l'extrémité proximale du corps (1) du tampon.

28. Procédé de fabrication d'un tampon hygiénique, comprenant les étapes consistant à : injecter une substance hydrophobe solide, ayant un point de fusion supérieur à 37°C, dans l'épaisseur d'une bande de matière absorbante, et former ladite bande de matière absorbante en un corps d'un tampon.

29. Procédé selon la revendication 28, **caractérisé en ce que** la substance hydrophobe est injectée de façon discontinue.

30. Procédé selon la revendication 28 à 29, **caractérisé en ce qu'**il comprend en outre une étape consistant à plier et éventuellement comprimer la bande de matière absorbante, suivie d'une étape consistant à chauffer ladite bande de matière absorbante à une température supérieure au point de fusion de la substance hydrophobe, en liant ensemble deux ou plusieurs parties de ladite bande contenant ladite substance hydrophobe.

31. Procédé selon l'une quelconque des revendications 28 à 30, **caractérisé en ce que** la substance hydrophobe est choisie dans le groupe constitué d'huiles et de graisses hydrogénées et non hydrogénées.

32. Procédé selon l'une quelconque des revendications 28 à 30, **caractérisé en ce que** la substance hydrophobe est choisie dans le groupe constitué de cires naturelles et synthétiques, dans lequel la substance hydrophobe est de préférence de la cire d'abeille ou de la cire de carnauba.

33. Procédé selon l'une quelconque des revendications 28 à 30, **caractérisé en ce que** la substance hydrophobe est de la vaseline hydrogénée.
